# EUROPEAN PATENT APPLICATION

(11) **EP 3 087 976 A1**
(43) Date of publication of application: **02.11.2016**
(21) Application number: 14873358.7
(22) Date of filing: 26.05.2014
(51) Int. Cl.: A61K 9/20, A61K 31/165, A61K 47/38, A61K 47/32, A61P 25/24, A61P 25/22

(54) **STABLE CRYSTAL X-FORM AGOMELATINE TABLET AND PREPARATION METHOD THEREOF**

(30) Priority: 23.12.2013 CN 201310712876
(71) Applicant: Tianjin Taipu Pharmaceutical Science & Technology Development Co., Ltd., Tianjin 300193 (CN)
(72) Inventor: ZHOU, Shiwang, Tianjin 300042 (CN); DAI, Yi, Tianjin 300168 (CN); AN, Shizhi, Tianjin 300193 (CN); ZHAO, Jian, Tianjin 300190 (CN)
(74) Representative: Zwicker, Jörk
(86) International application number: PCT/CN2014/000526
(87) International publication number: WO 2015/096186

(57) **Abstract**

Disclosed are a stable crystal X-form agomelatine tablet and a preparation method thereof. The method comprises the following steps: selecting one or more protective agents, adding the protective agents into pure water, stirring, heating the mixture to 35-40°C, dissolving them until the solution is clear, cooling the solution to room temperature, adding crystal X-form agomelatine, stirring the mixture until homogeneous, and obtaining the protective agents containing the crystal X-form agomelatine for use; and then, after mixing a part of the pharmaceutical excipient until homogeneous, again adding the protective agents containing the crystal X-form agomelatine, mixing and pelletizing them according to a wet method and drying to obtain particles containing the crystal X-form agomelatine; and finally, adding the other pharmaceutical excipients into the particles according to a proportion, mixing them until homogeneous and tabletting them.

## Description

### FIELD OF THE INVENTION

The present invention belongs to technical field of pharmaceutical preparations, and relates to a crystalline X-form agomelatine tablet and a manufacture method thereof.

### BACKGROUND OF THE INVENTION

Agomelatine is a melatonin drug for mental diseases. As a melatonin analogue, agomelatine is not only the first melatonin receptor agonist, but also a 5-hydroxytryptamine 2C (5-HT2C) receptor antagonist. Animal tests and clinical research demonstrate that the drug has anti-depression, anti-anxiety, sleep rhythm-adjusting and circadian clock-adjusting effects, with less adverse reaction, no bad influence on sexual function and no withdrawal syndrome.

The first melatonin receptor agonist agomelatine (Valdoxan) is a melatonin analogue, and also a 5-hydroxytryptamine 2C (5-HT2C) receptor antagonist. Melatonin has an affinity Ki of 8.85×10⁻¹¹ and 2.63×10⁻¹¹ with its receptors MT1 and MT2, respectively. Agomelatine, very similar to melatonin, also has high affinity with clonal human melatonin receptors MT1 and MT2 (Ki is 6.15×10⁻¹¹ and 2.68×10⁻¹¹, respectively). The clinical study shows that agomelatine has a better curative effect on the patients with depression, and the adverse reaction is very little.

The exact mechanism for agomelatine against depression is not clear yet. 5-HT2C receptor blocking agent alone does not exhibit an antidepressant effect. Agomelatine can block 5-HT2C receptor. However, animal tests show that melatonin also has a small antidepressant effect, and studies show that stress is related with melatonin secretion, but no evident antidepressant effect is observed in a human body after administration of melatonin.

Another study shows that the mechanism for agomelatine against depression is likely be associated with increased plasticity of hippocampus neurons and neuronal hyperplasia. The proliferation, regeneration and death of brain nerve cells of an adult rat were detected by an immunostaining method, and the result showed that long-term (three weeks) administration of agomelatine could increase cell proliferation and neuronal regeneration in hippocampal ventral dentate gyrus, which region is associated with emotional response. However, during the acute or subacute administration (4 hours or nine weeks), no similar situation occurred. Prolonging the time for administration, cell proliferation and neuronal regeneration occurred in the entire dentate gyrus region, which implied that agomelatine could increase hippocampal neurogenesis to different degrees, resulting in new granulosa cells.

Agomelatine is developed by Servier Company, and currently has been available on the market. It has a chemical structure below:

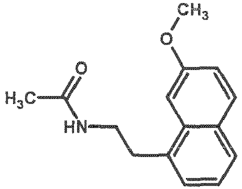

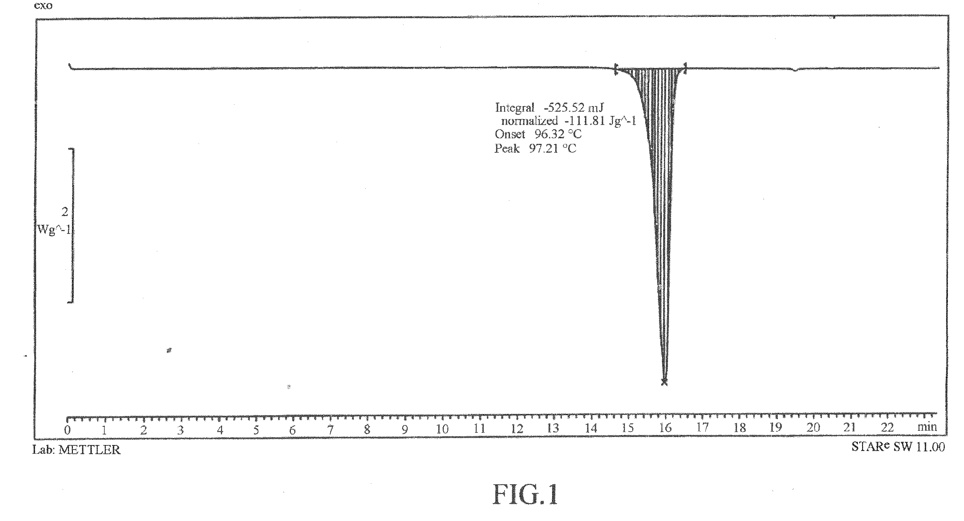

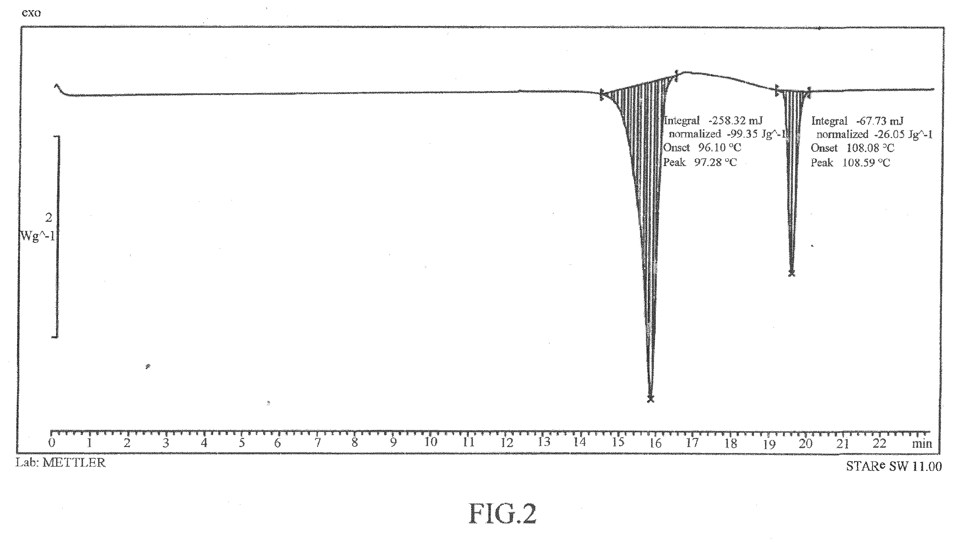

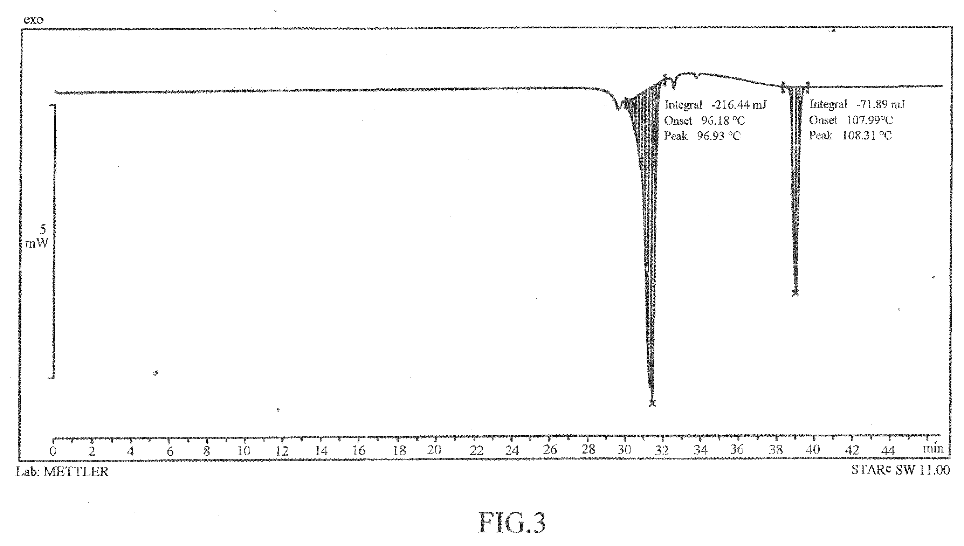

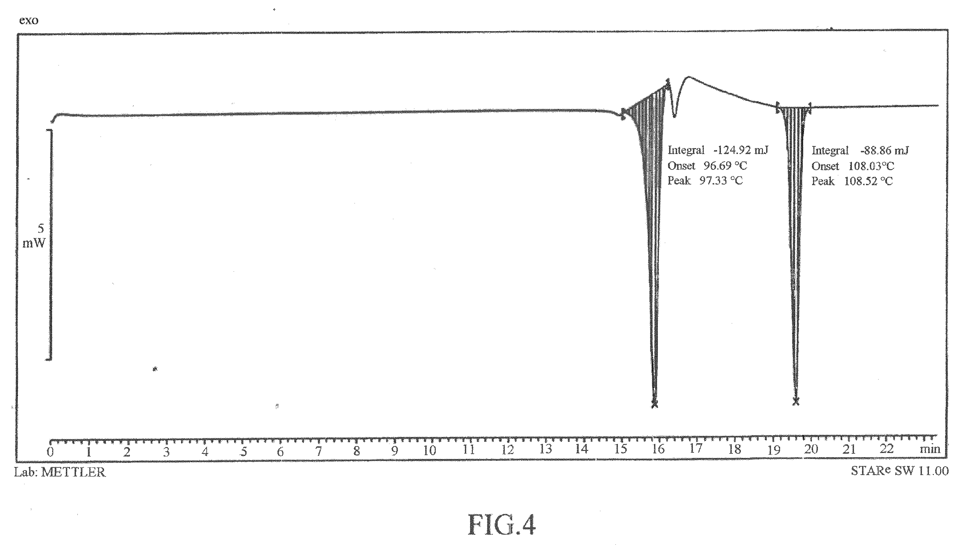

A plurality of crystal forms of agomelatine, such as I, II, III, IV, V and X, have been discovered. Agomelatine tablet is a commonly-used dosage form in clinic, but there exist the following difficulties in the manufacture of crystalline X-form agomelatine tablet.
(1) The crystalline X-form agomelatine raw material is sensitive to pulverization, grinding, pressure, heat and the like, and transformation thereof to II-form crystals occurs to different degrees. Change of the crystalline X-form raw material in pulverization, grinding, and tabletting (at a pressure of 10 kg) was detected by DSC (see figures 1-4).

| items | Crystalline X-form | Pulverization | Grinding | Tabletting |
|---|---|---|---|---|
| X-form crystal purity (%) detected by DSC | 100% | 79.4% | 75.1% | 57.7% |

The result showed that the crystalline X-form agomelatine raw material has a significant change in crystal form during the processes of pulverization, grinding and tabletting, which would be transformed into II-form crystals.
(2) Choice of adjuvant is limited: common adjuvant such as microcrystalline cellulose and pregelatinized starch cannot be used, mainly because the above adjuvant can accelerate transformation of agomelatine in crystal form to II-form crystals.

Accordingly, none of conventional granulating and tabletting processes can ensure the stability of X-form crystals. The crystal form varies instantly when the raw material and adjuvant are tabletted directly or dry granulated and tabletted. The transformation will be more remarkable in the case of a common wet granulation process.

At first, the patent application CN200510071611.6 filed by Sevier Lab in France involved a synthesis process of II-form crystals and a pharmaceutical composition, and was allowed in China in 2007, in which the crystalline II-form agomelatine has been claimed. Chinese patent CN101781226A recites a preparation method of the X-form crystals. Crystalline X-form agomelatine prepared by this method is very prone to transformation to the II form in the actual tablet manufacture process, and it is possible to further transform to the II form in the further test on stability (the sample tablets manufactured by us were subjected to powder diffraction examination after being accelerated for two months, and the raw material was almost completely converted to the II form).

Changes in crystal forms, on the one hand may result in infringement, and on the other hand may further lead to inconsistency in bioavailability of drugs. Therefore, it is very important to solve the problem of stability of crystal forms during the processes of the tablet manufacture and preservation.

### DESCRIPTION OF THE INVENTION

An object of the present invention is to overcome disadvantages and deficiencies of the prior art by providing a novel crystalline X-form agomelatine tablet and a manufacture method of such tablet. To achieve this object, the present invention provides the following technical solution.

A stable crystalline X-form agomelatine tablet is characterized in that it is composed of crystalline X-form agomelatine raw material, a protective agent, and a pharmaceutical adjuvant, wherein the weight ratio of the crystalline X-form agomelatine raw material, the protective agent, and the pharmaceutical adjuvant is 1:0.1-1:0.1-10; and the protective agent is one or more of polyvinylpyrrolidone, hydroxypropyl methyl cellulose and hydroxypropyl cellulose.

In the crystalline X-form agomelatine tablet according to the present invention, the crystalline X-form agomelatine raw material refers to an agomelatine raw material in which X-form crystals account for at least 85%, preferably at least 95%.

A preferred crystalline X-form agomelatine tablet according to the present invention consists of raw materials, based on the following weight parts:

| | |
|---|---|
| the crystalline X-form agomelatine | 1 |
| pure water | 0.5-10 |
| the protective agent | 0.1-1 |
| the pharmaceutical adjuvant | 0.1-10 |

wherein the protective agent is one or more of polyvinylpyrrolidone, hydroxypropyl methyl cellulose and hydroxypropyl cellulose; and the pharmaceutical adjuvant is lactose, crosslinked sodium carboxymethyl cellulose, crosslinked polyvinylpyrrolidone, stearic acid, magnesium stearate or silica.

The present invention further discloses a method for manufacturing the crystalline X-form agomelatine tablet, which comprises the following steps:
(a) choosing and adding to pure water one or more protective agents, stirring, heating to 35°C to 40°C and dissolving it till the solution is clear, then cooing to room temperature, adding the crystalline X-form agomelatine, and stirring uniformly to obtain a protective agent(s) containing the crystalline X-form agomelatine for use; wherein the weight ratio of the crystalline X-form agomelatine raw material to the protective agent(s) is 1:0.1-1; and water is added in an amount 0.5 to 4 times of the weight of the crystalline X-form agomelatine;
(b) mixing a part of pharmaceutical adjuvant uniformly, adding thereto the protective agent(s) containing the crystalline X-form agomelatine, then mixing and granulating to obtain granules containing the crystalline X-form agomelatine; wherein the part of pharmaceutical adjuvant is lactose, crosslinked sodium carboxymethyl cellulose or crosslinked polyvinylpyrrolidone; and
(c) adding the remaining pharmaceutical adjuvant in proportion, mixing uniformly and tabletting; wherein the protective agent is one or more of polyvinylpyrrolidone, hydroxypropyl methyl cellulose and hydroxypropyl cellulose.

The crystalline X-form agomelatine raw material of the present invention refers to an agomelatine raw material in which X-form crystals account for at least 85%, preferably at least 95%.

The protective agent in the present invention is one or more of hydroxypropyl methyl cellulose, hydroxypropyl cellulose, polyvinylpyrrolidone k30, and polyvinylpyrrolidone k90. The protective agent has a concentration generally in a range of between 5 and 40%, preferably between 10 and 30% (w/w), e.g., 5-20% of hydroxypropyl methyl cellulose, 5-20% of hydroxypropyl cellulose, 5-20% of polyvinylpyrrolidone k30 or 5-20% of polyvinylpyrrolidone k90.

The pharmaceutical adjuvant in the present invention is lactose, crosslinked sodium carboxymethyl cellulose, crosslinked polyvinylpyrrolidone, stearic acid, magnesium stearate or silica.

The present invention mainly chooses pure water as a solvent, and one ore more protective agents, such as polyvinylpyrrolidone, hydroxypropyl methyl cellulose and hydroxypropyl cellulose, are added. The mixture is stirred, heated to 35 to 40°C, dissolved till the solution is clear, and cooled to room temperature. Then, the crystalline X-form agomelatine is added, and stirred uniformly to obtain a protective agent(s) containing the crystalline X-form agomelatine for use. Subsequently, a part of pharmaceutical adjuvant, such as lactose, crosslinked sodium carboxymethyl cellulose or crosslinked polyvinylpyrrolidone, is mixed uniformly, and then the protective agent(s) containing the crystalline X-form agomelatine is added. The resulting mixture is subjected to wet mixing and granulating, and drying, thereby to obtain granules containing the crystalline X-form agomelatine. Finally, the remaining pharmaceutical adjuvant is added in proportion, mixed uniformly and tabletted.

In a preferred embodiment of the present invention, the crystalline X-form agomelatine (with a content of 85% or more) is sieved for use. Hydroxypropyl methyl cellulose or polyvinylpyrrolidone k90 is dissolved in water (about 40°C) under stirring, cooled to room temperature, added with the crystalline X-form agomelatine, and stirred uniformly to obtain a protective agent containing agomelatine for use. Subsequently, lactose and a part (1/2) of crosslinked sodium carboxymethyl cellulose are added to a wet mixing and granulating machine and mixed uniformly therein, and then the protective agent containing agomelatine is added. The mixture is granulated with an oscillating granulator, dried in a fluidized bed, and finished. The yield is calculated. The remaining pharmaceutical adjuvant is then added in proportion, mixed uniformly and tabletted.

In another preferred embodiment of the present invention, pure water is chosen as a solvent, and the crystalline X-form agomelatine (with a content of 99%) is sieved for use. Hydroxypropyl methyl cellulose and polyvinylpyrrolidone k30 are dissolved in water (about 40°C) under stirring, cooled to room temperature, added with the crystalline X-form agomelatine, and stirred uniformly to obtain a protective agent containing crystalline X-form agomelatine. Subsequently, lactose and a part (1/2) of crosslinked polyvinylpyrrolidone are added to a wet mixing and granulating machine and mixed uniformly therein, and then the protective agent containing the crystalline X-form agomelatine is added. The mixture is made into a soft material, granulated with an oscillating granulator, dried in a fluidized bed, and finished. The remaining pharmaceutical adjuvant is added in proportion, mixed uniformly and tabletted.

In still another preferred embodiment of the present invention, pure water is chosen as a solvent, and the crystalline X-form agomelatine (with a content of 95% or more) is sieved for use. Hydroxypropyl cellulose, hydroxypropyl methyl cellulose and polyvinylpyrrolidone k30 are dissolved in water (about 40°C) under stirring, cooled to room temperature, added with the crystalline X-form agomelatine, and stirred uniformly to obtain a protective agent containing crystalline X-form agomelatine. Subsequently, lactose and a part (1/2) of crosslinked polyvinylpyrrolidone are added to a wet mixing and granulating machine and mixed uniformly therein, and then the protective agent containing the crystalline X-form agomelatine is added. The mixture is made into a soft material, granulated with an oscillating granulator, dried in a fluidized bed, and finished. The remaining pharmaceutical adjuvant is added in proportion, mixed uniformly and tabletted. To this end, the present invention emphasizes on the following key issues:

### (1) Choice of the solvent

The crystalline X-form agomelatine is almost insoluble in water, but very soluble in methanol, ethanol, acetonitrile, DMSO, etc. which further leads to change in crystal forms. Hence, it is most preferred to prepare in water, and an optimal amount of the water added is about 0.5 to 4 times of the raw material.

### (2) Choice of the pharmaceutical adjuvant

The crystalline X-form agomelatine is sensitive to heat and pressure, and thus unstable under conditions of high temperature and high pressure. To this end, the inventors investigated mixing of adjuvant, such as lactose, mannitol, calcium hydrogen phosphate, microcrystalline cellulose, pregelatinized starch, polyethylene glycol 4000, polyvinyl pyrrolidone k30, hydroxypropyl methyl cellulose, hydroxypropyl cellulose, sodium carboxymethylstarch, crosslinked sodium carboxymethyl cellulose, crosslinked polyvinylpyrrolidone, magnesium stearate, stearic acid, silica, talc or the like, with the crystalline X-form agomelatine (at a ratio of 1:1). After the crystalline X-form agomelatine raw materials were placed simultaneously under conditions of a high temperature of 60°C, a high humidity RH92.5%, and an illuminance of 4500±500Lx for 15 days, DSC was adopted to detect change in the crystal forms.

Results: the pregelatinized starch interfered the detection, and the microcrystalline cellulose had remarkable effect of promoting crystal form transformation. With reference to the adjuvant used in marketed product, we chose lactose, polyvinylpyrrolidone, hydroxypropyl methyl cellulose, hydroxypropyl cellulose, sodium carboxymethyl starch, crosslinked polyvinylpyrrolidone, stearic acid, magnesium stearate, silica or the like as the adjuvant used in the tests.

### (3) Choice of the protective agent

Polyvinylpyrrolidone k30, hydroxypropyl methyl cellulose, polyethylene glycol and hydroxypropyl cellulose are chosen.

Test method: a proper amount of the protective agent was provided according to the prescription shown in the following table to prepare a 5% solution which was used as the protective agent for granulation. The crystalline X-form agomelatine was mixed with lactose and a part (1/2) of crosslinked polyvinylpyrrolidone. The resulting mixture was added to a (SHR-6 type) rapid wet granulator, mixed and granulated for 2 min, then transferred to an oscillating granulator for granulation (833 µm sieve), and dried in a (WBF-2 type) multifunctional fluidized bed (with an inlet air temperature of 45°C and a boiling bed temperature of 30°C). Yield was calculated. Afterward, a part (1/2) of crosslinked polyvinylpyrrolidone, magnesium stearate, stearic acid and silica were added according to the amount in the prescription, and the mixture was tabletted with a punch having Φ 7.5 mm. The tablets were subjected to DSC scanning, and purity of the X-form crystal was calculated by normalization.

**Formula table**

| Ingredients | Weight (g) |
|---|---|
| Crystalline X-form agomelatine | 25 |
| lactose | 92 |
| Protective agent | Pure water or 5% protective agent, 20 ml |
| Crosslinked polyvinylpyrrolidone | 9 |
| Magnesium stearate | 1.3 |
| Stearic acid | 2.6 |
| Silica | 0.3 |

The results are shown in the following table:

| items | Pure water | Polyvinylpyrrolidone k30 | Hydroxypropyl cellulose | Polyethylene glycol 4000 | Hydroxypropyl methyl cellulose |
|---|---|---|---|---|---|
| punty (%) of X-form crystals detected by DSC | 30% | 80% | 88% | 0 | 91% |

The results indicate that polyvinylpyrrolidone k30, hydroxpropyl cellulose and hydroxypropyl methyl cellulose all have a protective effect; and polyethylene glycol is effective in promoting transformation of crystal forms.

### 3.1 Tests on adding method of the protective agent

Method 1: the protective agent was mixed uniformly with agomelatine, and then lactose was added.
Method 2: agomelatine was mixed with lactose, and then the protective agent was added.

Polyvinylpyrrolidone k30 (with a concentration of 10%) was chosen as a protective agent, and tablets were manufactured according to the methods 1 and 2. The purity of X-form crystals in the tablets was detected with results shown below:

| Items | Method 1 | Method 2 |
|---|---|---|
| Purity (%) of X-form crystals detected by DSC | 92 | 65 |

The results indicate that method 1 achieved a better effect.

### 3.2 Tests on amount and usage of the protective agent

Test 1: polyvinylpyrrolidone k30 was chosen as a protective agent, and protective effects thereof in different concentrations were investigated. According to the above prescription, a proper amount of polyvinylpyrrolidone k30 was formulated into 5%, 10%, 15% and 20% solutions, and tablets were manufactured according the aforementioned method. The purity of X-form crystals in the tablets was detected with results below:

| | | | | |
|---|---|---|---|---|
| Concentration of polyvinylpyrrolidone k30 | 5% | 10% | 15% | 20% |
| Purity (%) of X-form crystals detected by DSC | 80 | 92 | 97 | 98 |

The results indicate that the change in crystal forms decreases with an increase in concentration of polyvinylpyrrolidone k30.
Test 2: hydroxypropyl cellulose was chosen as a protective agent, and protective effect thereof in different concentrations were investigated. According to the above prescription, a proper amount of hydroxypropyl cellulose was formulated into 5%, 10%, 15% and 20% solutions, and tablets were manufactured according the aforementioned method. The purity of X-form crystals in the tablets was detected with results below:

| | | | | |
|---|---|---|---|---|
| Concentration of hydroxypropyl cellulose | 5% | 10% | 15% | 20% |
| Purity (%) of X-form crystals detected by DSC | 88 | 97 | 97 | 97 |

The results indicate that the change in crystal forms decreases with an increase in concentration of hydroxypropyl cellulose.
Test 3: hydroxypropyl methyl cellulose was chosen as a protective agent, and protective effects thereof in different concentrations were investigated. According to the above prescription, a proper amount of hydroxypropyl methyl cellulose was formulated into 5%, 10%, 15% and 20% solutions, and tablets were manufactured according the aforementioned method. The purity of X-form crystals in the tablets was detected with results below:

| | | | | |
|---|---|---|---|---|
| Concentration of hydroxypropyl methyl cellulose | 5% | 10% | 15% | 20% |
| Purity (%) of X-form crystals detected by DSC | 91 | 97 | 98 | 98 |

The results indicate that hydroxypropyl methyl cellulose exhibits the strongest protective effect, and the X-form crystal purity of 90% or more could be achieved in a concentration of 5%; and the change in crystal forms decreases with an increase in concentration of hydroxypropyl methyl cellulose.
Test 4: polyvinylpyrrolidone k30 and hydroxypropyl cellulose were chosen as a protective agent, and protective effects thereof in different concentrations were investigated. According to the above prescription, a proper amount of polyvinylpyrrolidone k30 and hydroxypropyl cellulose (at a ratio of 1:1) was formulated into 5%, 10%, 15% and 20% solutions, and tablets were manufactured according the aforementioned method. The purity of X-form crystals in the tablets was detected with results below:

| | | | | |
|---|---|---|---|---|
| Concentration of the mixture of hydroxypropyl cellulose and polyvinylpyrrolidone k30 at a ratio of 1:1 | 5% | 10% | 15% | 20% |
| Purity (%) of X-form crystals detected by DSC | 97 | 98 | 99 | 99 |

The results indicate that the change in crystal forms decreases with an increase in concentration of polyvinylpyrrolidone k30 and hydroxypropyl cellulose.
Test 5: hydroxypropyl cellulose and hydroxypropyl methyl cellulose were chosen as a protective agent, and protective effects thereof in different concentrations were investigated. According to the above prescription, a proper amount of hydroxypropyl cellulose and hydroxypropyl methyl cellulose (at a ratio of 1:1) was formulated into 5%, 10%, 15% and 20% solutions, and tablets were manufactured according the aforementioned method. The purity of X-form crystals in the tablets was detected with results below:

| | | | | |
|---|---|---|---|---|
| Concentration of the mixture of hydroxypropyl methyl cellulose and hydroxypropyl cellulose at a ratio of 1:1 | 5% | 10% | 15% | 20% |
| Purity (%) of X-form crystals detected by DSC | 94 | 98 | 98 | 98 |

The results indicate that the change in crystal forms decreases with an increase in concentration of hydroxypropyl cellulose and hydroxypropyl methyl cellulose.
Test 6: hydroxypropyl methyl cellulose and polyvinylpyrrolidone k30 were chosen as a protective agent, and protective effects thereof in different concentrations were investigated. According to the above prescription, a proper amount of hydroxypropyl methyl cellulose and polyvinylpyrrolidone k30 (at a ratio of 1:1) was formulated into 5%, 10%, 15% and 20% solutions, and tablets were manufactured according the aforementioned method. The purity of X-form crystals in the tables was detected with results below:

| | | | | |
|---|---|---|---|---|
| Concentration of the mixture of hydroxypropyl methyl cellulose and polyvinylpyrrolidone k30 at a ratio of 1:1 | 5% | 10% | 15% | 20% |
| Purity (%) of X-form crystals detected by DSC | 91 | 99 | 100 | 100 |

The results indicate that the change in crystal form decreases with an increase in concentration of hydroxypropyl methyl cellulose and polyvinylpyrrolidone k30; and when the concentration reaches 15%, the crystalline X-form agomelatine almost has no change.

In summary, mixed protective agent achieves a best effect, where the most preferred is hydroxypropyl methyl cellulose and polyvinylpyrrolidone k30. Compared to a concentration of 20% (the viscosity is too high), a concentration of 15% makes the preparation of soft material easier, and is more beneficial to mass industrial production.

### 4. Test on in-vitro dissolution

Comparative test on *in-vitro* dissolution curve between (crystalline X-form) agomelatine solid formulation and a marketed product:
Dissolution was measured under the following conditions: operation was sequentially conducted according to a dissolution measurement method (the second method in the appendix XC of Part II of the Chinese pharmacopoeia, 2010 edition) at a rotation speed of 50 rpm with 900 ml of 0.1 mol/L hydrochloric acid as a solvent. 10 mL sample was collected at 5 min, 10 min, 15 min, 20 min, 30 min and 45 min, respectively, and liquid was supplemented in time. The samples were filtered and the filtrates were taken as the sample solutions to be tested. In addition, an appropriate amount of reference sample was weighed precisely, 95% ethanol solution was added to obtain a solution containing 1.25 mg agomelatine per 1 ml. 1 ml of the resulting solution was measured precisely, placed into a 50 ml graduated flask, diluted with 0.1 mol/l hydrochloric acid to the graduation line, and shaken to obtain a reference sample solution.

Absorbance of the sample solution to be tested and the reference sample solution was measured at the wavelength of 276 nm according to spectrophotography (the appendix IVA of Part II of the Chinese pharmacopoeia, 2010 edition), and the dissolution of each tablet was calculated according to an external standard method.

At the same time, the value of similarity factor f2 was calculated. The results are shown below.

**Dissolution (%) of self-made tablets in water**

| Time (min) | 1 | 2 | 3 | 4 | 5 | 6 | Average value |
|---|---|---|---|---|---|---|---|
| 5 | 34.6 | 34.4 | 33.6 | 35.7 | 31.7 | 34.0 | 34.0 |
| 10 | 62.5 | 63.3 | 61.1 | 64.5 | 63.6 | 63.5 | 63.1 |
| 15 | 80.7 | 80.4 | 79.5 | 81.2 | 81.0 | 81.2 | 80.7 |
| 20 | 87.3 | 88.5 | 87.9 | 90.9 | 89.9 | 89.1 | 88.9 |
| 30 | 93.4 | 94.6 | 94.0 | 95.4 | 94.4 | 94.1 | 94.3 |
| 45 | 95.5 | 94.9 | 95.0 | 96.0 | 95.4 | 95.1 | 95.3 |

**Dissolution (%) of self-made tablets in 0.1 mol/l hydrochloric acid**

| Time (min) | 1 | 2 | 3 | 4 | 5 | 6 | Average value |
|---|---|---|---|---|---|---|---|
| 5 | 33.3 | 34.4 | 33.8 | 34.3 | 32.8 | 34.6 | 33.9 |
| 10 | 64.5 | 66.1 | 62.4 | 66.9 | 64.1 | 64.6 | 64.8 |
| 15 | 79.4 | 82.0 | 81.0 | 83.9 | 83.4 | 83.8 | 82.3 |
| 20 | 87.7 | 89.8 | 87.0 | 90.6 | 90.3 | 89.5 | 89.1 |
| 30 | 93.6 | 94.6 | 93.9 | 95.5 | 95.7 | 95.9 | 94.9 |
| 45 | 97.1 | 95.8 | 95.2 | 96.8 | 97.2 | 95.8 | 96.3 |

**Dissolution (%) of self-made tablets in acetate buffer with pH of 4.5**

| Time (min) | 1 | 2 | 3 | 4 | 5 | 6 | Average value |
|---|---|---|---|---|---|---|---|
| 5 | 39.4 | 38.0 | 37.0 | 37.3 | 37.0 | 36.7 | 37.6 |
| 10 | 66.3 | 65.6 | 67.5 | 64.8 | 64.5 | 66.6 | 65.9 |
| 15 | 81.1 | 81.7 | 81.9 | 79.9 | 81.1 | 83.0 | 81.5 |
| 20 | 90.3 | 90.5 | 91.9 | 88.4 | 88.8 | 90.5 | 90.1 |
| 30 | 94.5 | 96.3 | 96.8 | 94.4 | 94.1 | 95.3 | 95.2 |
| 45 | 97.3 | 98.4 | 99.3 | 96.7 | 98.2 | 97.3 | 97.9 |

**Dissolution (%) of self-made tablets in phosphate buffer with pH of 6.8**

| Time (min) | 1 | 2 | 3 | 4 | 5 | 6 | Average value |
|---|---|---|---|---|---|---|---|
| 5 | 39.1 | 34.8 | 34.4 | 33.5 | 34.9 | 35.4 | 35.4 |
| 10 | 62.6 | 63.2 | 62.7 | 62.2 | 64.3 | 63.5 | 63.1 |
| 15 | 80.4 | 80.1 | 77.7 | 76.8 | 78.6 | 78.2 | 78.6 |
| 20 | 89.8 | 88.6 | 88.3 | 85.5 | 89.1 | 87.3 | 88.1 |
| 30 | 95.4 | 93.4 | 91.2 | 93.0 | 94.1 | 92.9 | 93.3 |
| 45 | 97.9 | 96.4 | 95.8 | 96.1 | 96.4 | 93.6 | 96.0 |

**Dissolution (%) of self-made tablets in 0.5% SDS solution**

| Time (min) | 1 | 2 | 3 | 4 | 5 | 6 | Average value |
|---|---|---|---|---|---|---|---|
| 5 | 77.7 | 75.5 | 76.4 | 75.8 | 75.6 | 73.9 | 75.8 |
| 10 | 96.8 | 97.1 | 97.3 | 95.8 | 95.7 | 96.8 | 96.6 |
| 15 | 99.7 | 100.1 | 99.2 | 98.3 | 98.7 | 98.8 | 99.1 |
| 20 | 101.4 | 99.3 | 98.8 | 99.4 | 98.9 | 99.4 | 99.5 |
| 30 | 100.9 | 99.1 | 98.9 | 98.7 | 100.0 | 100.3 | 99.6 |
| 45 | 101.3 | 100.0 | 99.8 | 100.6 | 99.5 | 99.5 | 100.1 |

**Dissolution (%) of the marketed product in water**

| Time (min) | 1 | 2 | 3 | 4 | 5 | 6 | Average value |
|---|---|---|---|---|---|---|---|
| 5 | 42.5 | 46.3 | 43.4 | 45.0 | 44.1 | 42.9 | 44.0 |
| 10 | 80.7 | 77.1 | 79.5 | 78.8 | 77.5 | 76.3 | 78.3 |
| 15 | 92.4 | 91.2 | 92.8 | 92.2 | 94.1 | 92.5 | 92.5 |
| 20 | 95.5 | 95.1 | 94.3 | 95.5 | 94.8 | 95.6 | 95.1 |
| 30 | 99.4 | 98.7 | 98.2 | 99.2 | 99.4 | 96.8 | 98.6 |
| 45 | 98.7 | 99.9 | 99.6 | 98.8 | 99.1 | 98.1 | 99.1 |

**Dissolution (%) of the marketed product in 0.1 mol/l hydrochloric acid**

| Time (min) | 1 | 2 | 3 | 4 | 5 | 6 | Average value |
|---|---|---|---|---|---|---|---|
| 5 | 26.9 | 24.2 | 25.2 | 23.1 | 28.5 | 26.3 | 25.7 |
| 10 | 67.6 | 64.5 | 63.6 | 62.4 | 65.0 | 63.9 | 64.5 |
| 15 | 86.3 | 85.2 | 85.6 | 82.6 | 85.9 | 86.4 | 85.3 |
| 20 | 91.2 | 89.3 | 91.3 | 89.8 | 90.7 | 91.1 | 90.6 |
| 30 | 97.2 | 94.5 | 95.3 | 94.8 | 94.4 | 95.0 | 95.2 |
| 45 | 98.5 | 95.2 | 95.9 | 96.4 | 97.0 | 97.0 | 96.7 |

**Dissolution (%) of the marketed product in a buffer with pH of 4.5**

| Time (min) | 1 | 2 | 3 | 4 | 5 | 6 | Average value |
|---|---|---|---|---|---|---|---|
| 5 | 32.4 | 31.6 | 29.5 | 33.0 | 30.0 | 28.7 | 30.8 |
| 10 | 71.6 | 70.7 | 68.7 | 69.3 | 69.4 | 70.2 | 70.0 |
| 15 | 87.8 | 87.5 | 85.7 | 86.5 | 87.9 | 88.6 | 87.3 |
| 20 | 94.6 | 93.7 | 92.9 | 93.4 | 94.8 | 93.5 | 93.8 |
| 30 | 98.5 | 97.3 | 97.2 | 97.1 | 96.7 | 97.3 | 97.3 |
| 45 | 100.3 | 99.2 | 98.6 | 98.2 | 98.5 | 98.5 | 98.9 |

**Dissolution (%) of the marketed product in a buffer with pH of 6.8**

| Time (min) | 1 | 2 | 3 | 4 | 5 | 6 | Average value |
|---|---|---|---|---|---|---|---|
| 5 | 37.2 | 34.9 | 36.2 | 35.1 | 34.6 | 35.9 | 35.6 |
| 10 | 77.0 | 70.4 | 72.8 | 75.2 | 71.2 | 73.6 | 73.4 |
| 15 | 88.7 | 88.2 | 87.9 | 87.4 | 89.0 | 88.5 | 88.3 |
| 20 | 95.0 | 93.3 | 94.8 | 92.2 | 94.3 | 92.1 | 93.6 |
| 30 | 96.7 | 96.4 | 96.9 | 96.3 | 97.2 | 95.9 | 96.6 |
| 45 | 98.7 | 98.1 | 97.8 | 98.4 | 97.8 | 97.4 | 98.0 |

**Dissolution (%) of the marketed product in 0.5% sodium dodecyl sulfate solution**

| Time (min) | 1 | 2 | 3 | 4 | 5 | 6 | Average value |
|---|---|---|---|---|---|---|---|
| 5 | 49.3 | 50.1 | 53.3 | 52.5 | 51.7 | 48.7 | 51.0 |
| 10 | 97.9 | 96.4 | 97.4 | 95.9 | 98.9 | 97.1 | 97.3 |
| 15 | 99.5 | 99.7 | 99.4 | 99.4 | 101.3 | 99.7 | 99.8 |
| 20 | 100.8 | 100.3 | 99.2 | 100.3 | 101.1 | 101.2 | 100.5 |
| 30 | 100.7 | 100.1 | 100.9 | 100.3 | 100.1 | 101.0 | 100.5 |
| 45 | 100.8 | 100.7 | 99.9 | 100.5 | 101.1 | 101.5 | 100.7 |

Result of comparison between a dissolution curve of the self-made product and that of the marketed product

| dissolution media | water | 0.1 mol/LHCl solution | Acetate buffer with pH 4.5 | Phosphate buffer with pH 6.8 |
|---|---|---|---|---|
| similarity factor f₂ | 51.2 | 71.4 | 68.0 | 59.9 |

The results show that the f₂ values of the self-made product and marketed product in the above various media are all greater than 50, which demonstrates the in-vitro dissolutions of both are very similar.

### 5. Investigation on stability

Referring to the aforementioned test 6, tablets were manufactured according to formulation and process with hydroxyproplyl methyl cellulose and polyvinylpyrrolindone k30 in a concentration of 15% as a protective agent.

### 5.1 Stability in the process

Crystal form purity of the tablets was detected using DSC method with crystal forms and relevant substances as evaluation indexes. Regarding detection of relevant substances, HPLC method was adopted using imported drug as a standard. The results are as follows:
Results of the X-form crystalline agomelatine tablet manufactured by the present invention

| Items | Relevant substances | Crystal form purity |
|---|---|---|
| Raw material | 0.42 | 100 |
| Granules containing raw material | 0.44 | 100 |
| tablet | 0.43 | 100 |

The results indicate that the crystal form and relevant substances almost have no change during the formulation process, and the process is good.

### 5.2 Test on influencing factors:

We investigated in combination with formulations of the present invention: the samples were allowed to stand in an open state for 30 days under the conditions of a high temperature of 60°C, a high humidity of RH 92%, and both high temperature and high humidity (40°C, RH75%), respectively. The results are as follows:

**Test results on influencing factors (%)**

| items | Crystal form purity of the raw material % | Tablet comprising a protective agent | |
|---|---|---|---|
| | | Crystal form purity % | Relevant substances % |
| 0 day | 100 | 100 | 0.41 |
| 10 days at a high temperature | 68 | 93 | 0.42 |
| 10 days at a high humidity | 85 | 98 | 0.42 |
| 10 days at both high temperature and high humidity | 57 | 86 | 0.43 |

The results indicate that relevant substances are not increased, and the stability of crystal form in the crystalline X-form formulation is remarkably improved in comparison with pure raw material.

### 5.3 Accelerated test:

The sample was packaged with a polyethylene bottle to which a drier was added. The sample was allowed to stand under the conditions of RH 75% and 40°C and conditions of RH60% and 30°C. Crystal form was taken as an evaluation index. The results are as follows:

| Time | Crystal form purity % under conditions of RH75% and 40°C | Crystal form purity % under conditions of RH60% and 30°C |
|---|---|---|
| 0 day | 100% | 100% |
| Accelerated by 1 month | 85 | 100 |
| Accelerated by 2 months | 78 | 100 |
| Accelerated by 3 months | 70 | 100 |
| Accelerated by 6 months | 54 | 100 |

The results indicate that change in the crystal form occurs under conditions of RH75% and 40°C, but the crystal form is stable under conditions of RH60% and 30°C, which implies that the tablet should be stored in the shade.

The crystalline X-form agomelatine tablet disclosed in the present invention has the following characteristics in comparison with the prior art:
(1) The protective agent(s) used in the present invention is/are selected from commonly-used pharmaceutical adjuvant in formulations, such as hydroxypropyl cellulose, hydroxypropyl methyl cellulose or polyvinylpyrrolidone. The method of adding the protective agent comprises fully and uniformly mixing and stirring the crystalline X-form agomelatine with an aqueous solution of a protective agent in a certain concentration to obtain a protective agent containing the crystalline X-form agomelatine, then mixing it with other pharmaceutical adjuvant(s), granulating, and finally obtaining agomelatine tablets that ensure the X-form crystals do not have any change.
(2) The crystalline X-form agomelatine tablet manufactured according to the present invention can sufficiently ensure the X-form crystals do not change in the manufacture of the tablet.
(3) The process of manufacturing the tablet disclosed in the present invention can completely satisfy the requirements of large-scale industrial production.
(4) The crystal form and relevant substances in the tablet show good stability in the process of manufacturing the tablet disclosed in the present invention.

### DESCRIPTIONS OF THE FIGURES

**Figure 1** shows the DSC curve of crystalline X-form AG raw material;
**Figure 2** shows the DSC curve of crystalline X-form AG raw material after pulverization;
**Figure 3** shows the DSC curve of crystalline X-form AG raw material after grinding;
**Figure 4** shows the DSC curve of raw material after tabletting;

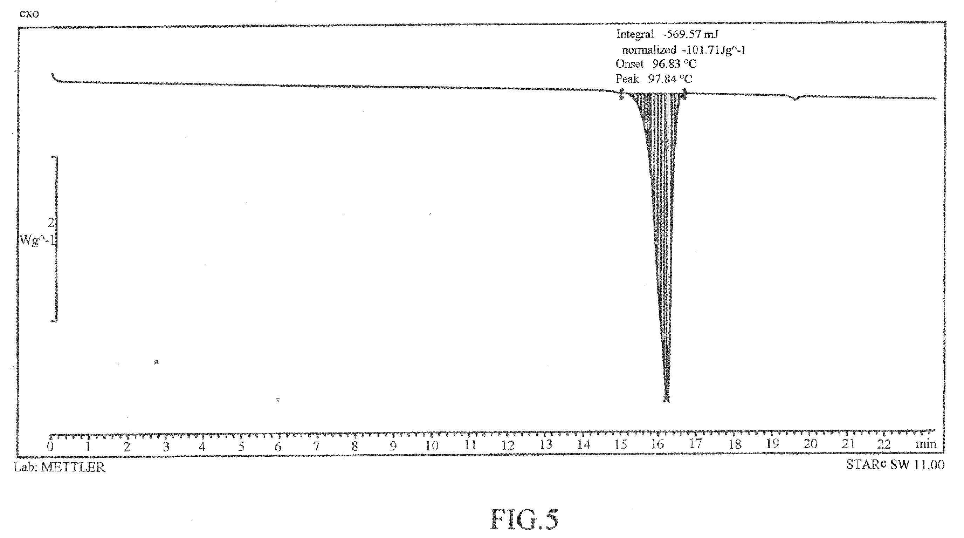

**Figure 5** shows the DSC curve of protected crystalline X-form AG raw material after tabletting;

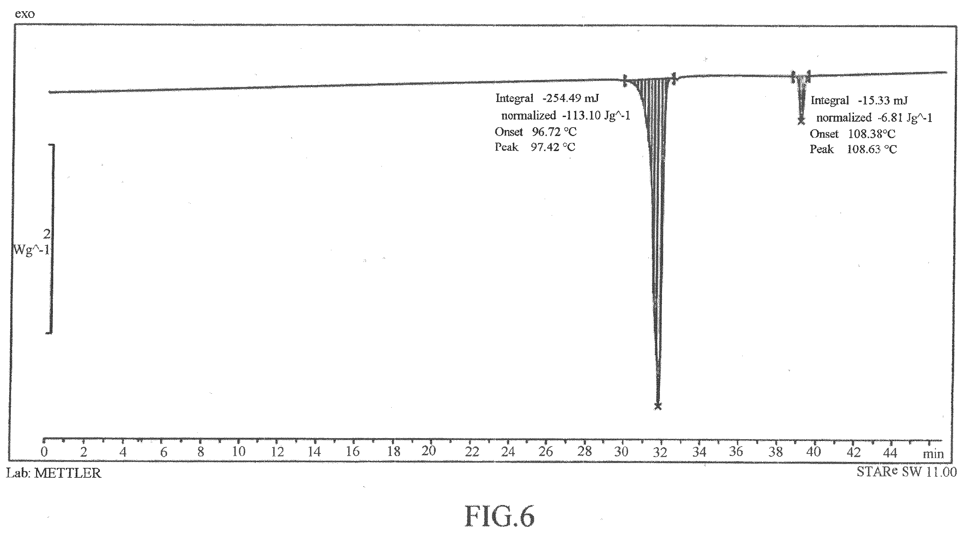

**Figure 6** shows the DSC curve of crystalline X-form AG raw material (containing mixed crystals);

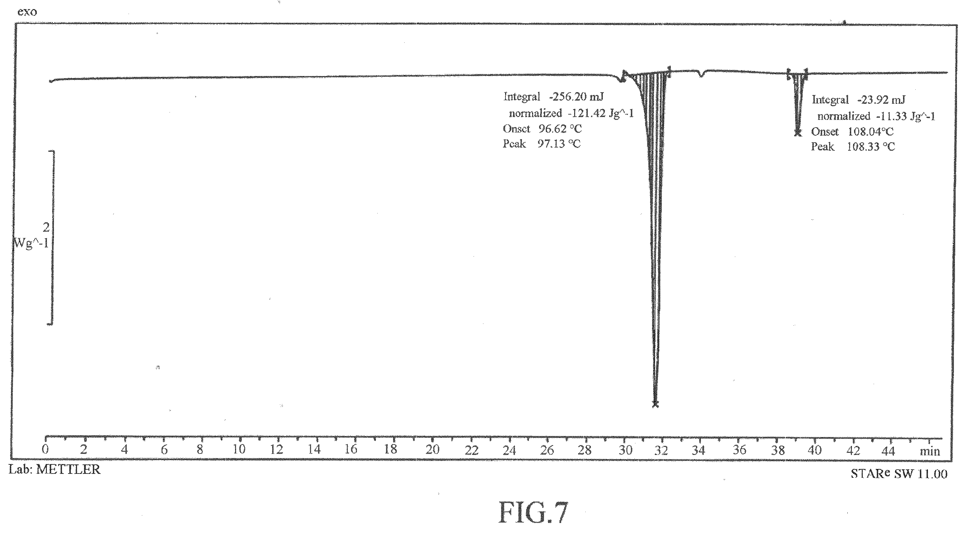

**Figure 7** shows the DSC curve of protected crystalline X-form AG raw material (containing mixed crystals) after tabletting;

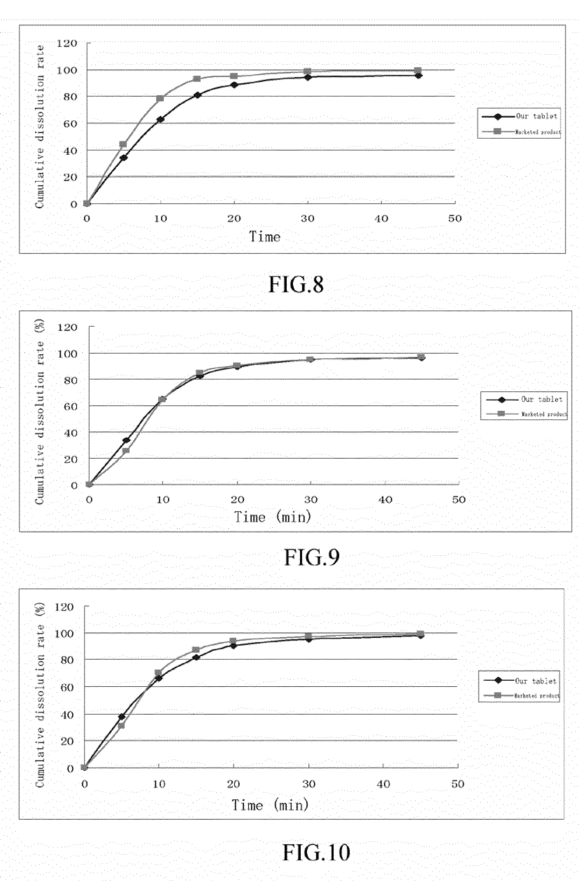

**Figure 8** shows comparison of dissolution curves in water;
**Figure 9** shows comparison of dissolution curves in 0.1 mol/L hydrochloric acid;
**Figure 10** shows comparison of dissolution curves in acetate buffer with pH 4.5;

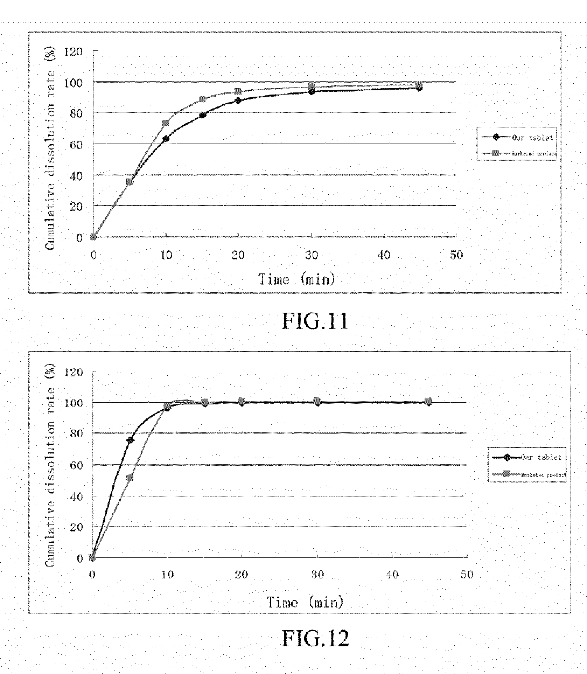

**Figure 11** shows comparison of dissolution curves in phosphate buffer with pH 6.8; and
**Figure 12** shows comparison of dissolution curves in 0.5% solution of sodium dodecyl sulfate.

### DETAILED DESCRIPTION OF EMBODIMENTS

For simplicity and clarity, the description of well known techniques is omitted below so as to avoid the influence of those unnecessary details on the description of the present technical solutions. The present invention is further explained in combination with the following examples. With respect to the preparation of agomelatine (with a content of X-form crystals of 85% or more), please refer to Chinese patent CN101781226A; and other adjuvants used are all commercially available.

### Example 1

| | |
|---|---|
| Agomelatine (X-form crystal 99%) | 25 g |
| Water | 20 ml |
| Lactose | 102 g |
| Hydroxypropyl methyl cellulose | 3 g |
| Polyvinylpyrrolidone k30 | 3 g |
| Crosslinked polyvinylpyrrolidone | 13 g |
| Magnesium stearate | 1.3 g |
| Stearic acid | 2.6 g |
| Silica | 0.3 g |

Process: according to the above weight, the crystalline X-form agomelatine was sieved for use. Hydroxypropyl methyl cellulose and polyvinylpyrrolidone k30 were stirred and dissolved in water, cooled to room temperature, added with the crystalline X-form agomelatine, and stirred uniformly to give a protective agent containing the crystalline X-form agomelatine for use. Subsequently, the protective agent containing the crystalline X-form agomelatine was added to a mixing and granulating machine containing lactose and a part (1/2) of crosslinked polyvinylpyrrolidone, subjected to wet granulation for 2 min, and then granulated with an oscillating granulator (833 µm sieve). The obtained wet granulates was dried in a fluidized bed (an inlet air temperature 45°C, and a boiling bed temperature 30°C) with moisture content controlled at about 2%, and finished. The yield was calculated. The remaining other adjuvant was added and mixed uniformly. The resultant material was tabletted with a punch having a diameter of 7.5 mm.

### Example 2

| | |
|---|---|
| Agomelatine (X-form crystal 90% or more) | 25 g |
| Water | 30 ml |
| Lactose | 102 g |
| Hydroxypropyl cellulose | 4.5 g |
| Polyvinylpyrrolidone k30 | 4.5 g |
| Crosslinked sodium carboxymethyl cellulose | 13 g |
| Magnesium stearate | 1.3 g |
| Stearic acid | 2.6 g |
| Silica | 0.3 g |

Process: according to the above weight, the crystalline X-form agomelatine was sieved for use. Hydroxypropyl cellulose and polyvinylpyrrolidone k30 were stirred and dissolved in water, cooled to room temperature, added with the crystalline X-form agomelatine, and stirred uniformly to give a protective agent containing the crystalline X-form agomelatine for use. Subsequently, the protective agent containing the crystalline X-form agomelatine was added to a mixing and granulating machine containing lactose and a part (1/2) of crosslinked sodium carboxymethyl cellulose, subjected to wet granulation for 2 min, and then granulated with an oscillating granulator (833 µm sieve). The obtained wet granulates was dried in a fluidized bed (an inlet air temperature 45°C, and a boiling bed temperature 30°C) with moisture content controlled at about 2%, and finished. The yield was calculated. The remaining other adjuvant was added and mixed uniformly. The resultant material was tabletted with a punch having a diameter of 7.5 mm.

### Example 3

| | |
|---|---|
| Agomelatine (X-form crystal 85% or more) | 25 g |
| Water | 30 ml |
| Lactose | 102 g |
| Hydroxypropyl methyl cellulose | 4.5 g |
| Hydroxypropyl cellulose | 4.5 g |
| Crosslinked sodium carboxymethyl cellulose | 13 g |
| Magnesium stearate | 1.3 g |
| Stearic acid | 2.6 g |
| Silica | 0.3 g |

Process: according to the above weight, the crystalline X-form agomelatine was sieved for use. Hydroxypropyl methyl cellulose and polyvinylpyrrolidone k30 were stirred and dissolved in water, cooled to room temperature, added with the crystalline X-form agomelatine, and stirred uniformly to give a protective agent containing the crystalline X-form agomelatine for use. Subsequently, the protective agent containing the crystalline X-form agomelatine was added to a mixing and granulating machine containing lactose and a part (1/2) of crosslinked sodium carboxymethyl cellulose, subjected to wet granulation for 2 min, and then granulated with an oscillating granulator (833 µm sieve). The obtained wet granulates was dried in a fluidized bed (an inlet air temperature 45°C, and a boiling bed temperature 30°C) with moisture content controlled at about 2%, and finished. The yield was calculated. The remaining other adjuvant was added and mixed uniformly. The resultant material was tabletted with a punch having a diameter of 7.5 mm.

### Example 4

| | |
|---|---|
| Agomelatine (X-form crystal 90%) | 25 g |
| Water | 20 ml |
| Lactose | 99 g |
| Hydroxypropyl methyl cellulose | 9 g |
| Crosslinked polyvinylpyrrolidone | 13 g |
| Magnesium stearate | 1.3 g |
| Stearic acid | 2.6 g |
| Silica | 0.3 g |

Process: according to the above weight, the crystalline X-form agomelatine was sieved for use. Hydroxypropyl methyl cellulose was stirred and dissolved in water, cooled to room temperature, added with the crystalline X-form agomelatine, and stirred uniformly to give a protective agent containing the crystalline X-form agomelatine for use. Subsequently, the protective agent containing the crystalline X-form agomelatine was added to a mixing and granulating machine containing lactose and a part (1/2) of crosslinked polyvinylpyrrolidone, subjected to wet granulation for 2 min, and then granulated with an oscillating granulator (833 µm sieve). The obtained wet granulates was dried in a fluidized bed (an inlet air temperature 45°C, and a boiling bed temperature 30°C) with moisture content controlled at about 2%, and finished. The yield was calculated. The remaining other adjuvant was added and mixed uniformly. The resultant material was tabletted with a punch having a diameter of 7.5 mm.

### Example 5

| | |
|---|---|
| Agomelatine (X-form crystal 95%) | 25 g |
| Water | 20 ml |
| Lactose | 99 g |
| Polyvinylpyrrolidone k90 | 9 g |
| Crosslinked sodium carboxymethyl cellulose | 13 g |
| Magnesium stearate | 1.3 g |
| Stearic acid | 2.6 g |
| Silica | 0.3 g |

Process: according to the above weight, the crystalline X-form agomelatine was sieved for use. Polyvinylpyrrolidone k90 was stirred and dissolved in water, cooled to room temperature, added with the crystalline X-form agomelatine, and stirred uniformly to give a protective agent containing the crystalline X-form agomelatine for use. Subsequently, the protective agent containing the crystalline X-form agomelatine was added to a mixing and granulating machine containing lactose and a part (1/2) of crosslinked sodium carboxymethyl cellulose, subjected to wet granulation for 2 min, and then granulated with an oscillating granulator (833 µm sieve). The obtained wet granulates was dried in a fluidized bed (an inlet air temperature 45°C, and a boiling bed temperature 30°C) with moisture content controlled at about 2%, and finished. The yield was calculated. The remaining other adjuvant was added and mixed uniformly. The resultant material was tabletted with a punch having a diameter of 7.5 mm.

### Example 6

| | |
|---|---|
| Agomelatine (X-form crystal 99%) | 25 g |
| Water | 20 ml |
| Lactose | 99 g |
| Hydroxypropyl cellulose | 9 g |
| Crosslinked polyvinylpyrrolidone | 12 g |
| Magnesium stearate | 1.3 g |
| Stearic acid | 2.6 g |
| Silica | 0.3 g |

Process: according to the above weight, the crystalline X-form agomelatine was sieved for use. Hydroxypropyl cellulose was stirred and dissolved in water, cooled to room temperature, added with the crystalline X-form agomelatine, and stirred uniformly to give a protective agent containing the crystalline X-form agomelatine for use. Subsequently, the protective agent containing the crystalline X-form agomelatine was added to a mixing and granulating machine containing lactose and a part (1/2) of crosslinked polyvinylpyrrolidone, subjected to wet granulation for 2 min, and then granulated with an oscillating granulator (833 µm sieve). The obtained wet granulates was dried in a fluidized bed (an inlet air temperature 45°C, and a boiling bed temperature 30°C) with moisture content controlled at about 2%, and finished. The yield was calculated. The remaining other adjuvant was added and mixed uniformly. The resultant material was tabletted with a punch having a diameter of 7.5 mm.

### Example 7

| | |
|---|---|
| Agomelatine (X-form crystal 85% or more) | 25 g |
| Water | 20 ml |
| Lactose | 99 g |
| Hydroxypropyl methyl cellulose | 3 g |
| Hydroxypropyl cellulose | 3 g |
| Polyvinylpyrrolidone k30 | 3 g |
| Crosslinked polyvinylpyrrolidone | 12 g |
| Magnesium stearate | 1.3 g |
| Stearic acid | 2.6 g |
| Silica | 0.3 g |

Process: according to the above weight, the crystalline X-form agomelatine was sieved for use. Hydroxypropyl cellulose, hydroxypropyl methyl cellulose and polyvinylpyrrolidone k30 were stirred and dissolved in water, cooled to room temperature, added with the crystalline X-form agomelatine, and stirred uniformly to give a protective agent containing the crystalline X-form agomelatine for use. Subsequently, the protective agent containing the crystalline X-form agomelatine was added to a mixing and granulating machine containing lactose and a part (1/2) of crosslinked polyvinylpyrrolidone, subjected to wet granulation for 2 min, and then granulated with an oscillating granulator (833 µm sieve). The obtained wet granulates was dried in a fluidized bed (an inlet air temperature 45°C, and a boiling bed temperature 30°C) with moisture content controlled at about 2%, and finished. The yield was calculated. The remaining other adjuvant was added and mixed uniformly. The resultant material was tabletted with a punch having a diameter of 7.5 mm.

## Claims

1. A stable crystalline X-form agomelatine tablet, **characterized in that** it is composed of a crystalline X-form agomelatine raw material, a protective agent, and a pharmaceutical adjuvant, wherein the weight ratio of the crystalline X-form agomelatine raw material, the protective agent, and the pharmaceutical adjuvant is 1:0.1-1:0.1-10; wherein the protective agent is one or more of polyvinylpyrrolidone, hydroxypropyl methyl cellulose and hydroxypropyl cellulose.

2. The crystalline X-form agomelatine tablet according to claim 1, wherein the crystalline X-form agomelatine raw material refers to an agomelatine raw material in which X-form crystals account for at least 85%.

3. The crystalline X-form agomelatine tablet according to claim 1, wherein the crystalline X-form agomelatine raw material refers to an agomelatine raw material in which X-form crystals account for at least 95%.

4. The crystalline X-form agomelatine tablet according to claim 1, consisting of raw materials, based on the following weight parts:
| | |
|---|---|
| the crystalline X-form agomelatine | 1 |
| pure water | 0.5-10 |
| the protective agent | 0.1-1 |
| the pharmaceutical adjuvant | 0.1-10 |
wherein the protective agent is one or more of polyvinylpyrrolidone, hydroxypropyl methyl cellulose and hydroxypropyl cellulose; and the pharmaceutical adjuvant is lactose, crosslinked sodium carboxymethyl cellulose, crosslinked polyvinylpyrrolidone, stearic acid, magnesium stearate or silica.

5. A manufacture method of the crystalline X-form agomelatine tablet according to any one of claims 1 to 4, **characterized in that** it is carried out based on the following steps:
(a) choosing and adding to pure water one or more protective agents, stirring, heating to 35°C to 40°C and dissolving it till the solution is clear, cooing to room temperature, adding a crystalline X-form agomelatine, and stirring uniformly to obtain a protective agent(s) containing the crystalline X-form agomelatine for use; wherein the weight ratio of a crystalline X-form agomelatine raw material to the protective agent(s) is 1:0.1-1; and water is added in an amount 0.5 to 4 times of the weight of the crystalline X-form agomelatine;
(b) mixing a part of pharmaceutical adjuvant uniformly, adding thereto the protective agent(s) containing the crystalline X-form agomelatine, then mixing and granulating to obtain granules containing the crystalline X-form agomelatine; wherein the part of pharmaceutical adjuvant is lactose, crosslinked sodium carboxymethyl cellulose or crosslinked polyvinylpyrrolidone; and
(c) adding the remaining pharmaceutical adjuvant in proportion, mixing uniformly and tabletting; wherein the protective agent is one or more of polyvinylpyrrolidone, hydroxypropyl methyl cellulose and hydroxypropyl cellulose.

6. The manufacture method according to claim 5, wherein the crystalline X-form agomelatine raw material refers to an agomelatine raw material in which X-form crystals account for at least 85%.

7. The manufacture method according to claim 5, wherein the crystalline X-form agomelatine raw material refers to an agomelatine raw material in which X-form crystals account for at least 95%.

8. The manufacture method according to claim 5, wherein the protective agent is one or more of hydroxypropyl methyl cellulose, hydroxypropyl cellulose, polyvinylpyrrolidone k30, and polyvinylpyrrolidone k90.

9. The manufacture method according to claim 8, wherein the protective agent has a concentration of 5-40% (w/w).

10. The manufacture method according to claim 5, wherein the pharmaceutical adjuvant is lactose, crosslinked sodium carboxymethyl cellulose, crosslinked polyvinylpyrrolidone, stearic acid, magnesium stearate or silica.
